# EUROPEAN PATENT APPLICATION

(11) **EP 4 696 685 A1**
(43) Date of publication of application: **18.02.2026**
(21) Application number: 24195004.7
(22) Date of filing: 16.08.2024
(51) Int. Cl.: C07D 307/48

(54) **PROCESS FOR PRODUCING 5-(ALKOXYMETHYL)FURFURAL FROM A COMPOSITION COMPRISING CELLULOSE AND/OR STARCH OR SOURCE THEREOF**

(71) Applicant: Avantium Knowledge Centre B.V., 1014 BV Amsterdam (NL)
(72) Inventor: BUENO MORON, Jorge, 1014 BV Amsterdam (NL); GRUTER, Gerardus Johannes Maria, 1014 BV Amsterdam (NL); VAN KLINK, Gerardus Petrus Maria, 1014 BV Amsterdam (NL)
(74) Representative: Avantium Intellectual Property

(57) **Abstract**

A process for producing 5-(alkoxymethyl)furfural from a composition comprising cellulose and/or starch, or a source of cellulose or starch. The composition comprising cellulose or source thereof may be cotton, optionally mixed with man-made fibres such as polyester or polyamide fibres. The 5-(alkoxymethyl)furfural is preferably 5-(methoxymethyl)furfural or 5-(ethoxymethyl)furfural.

## Description

### Introduction

The present invention relates to a process for producing 5-(alkoxymethyl)furfural from a composition comprising cellulose and/or starch, or a source of cellulose or starch. The composition comprising cellulose or source thereof may be cotton, optionally mixed with man-made fibres such as polyester or polyamide fibres. The 5-(alkoxymethyl)furfural is preferably 5-(methoxymethyl)furfural or 5-(ethoxymethyl)furfural.

### Background of the invention

Patent application WO2023/166122 discloses a process in which a chemical building block is are prepared from fabric material containing cellulose fibres such as cotton next to man-made fibres. The building block that is produced by the method of this reference is 5-(chloromethyl)furfural (CMF). It is referred to that CMF obtained can be isolated or can be processed further in other chemical useful components, such as to 5-(alkoxymethyl)furfural, e.g. 5-(methoxymethyl)furfural (MMF) and 5-(ethoxymethyl)furfural (EMF), to HMF, HMF acetate (AcMF) or to methylfurfural (MF). This can be done with processes which are known as such by reacting CMF with methanol, ethanol, water, acetic acid or hydrogen, respectively.

This conversion of CMF into 5-(alkoxymethyl)furfural has been described by SB Onkarappa, Chemistry Select, 2019, 4, 5540-5543. In this reference, CMF is mixed with excess alcohol (e.g. methanol and ethanol) in a ratio CMF : alkanol of between 1 : 1 and 1 : 25 and subjected to a temperature of between room temperature and 50°C to give 5-(methoxymethyl)furfural (MMF) and 5-(ethoxymethyl)furfural (EMF).

The above reference achieves the conversion of CMF, apparently in isolated form, to MMF or EMF. The patent application referred to achieved production of CMF. However, the CMF is produced by the process of WO2023/166122 dissolved in an organic extraction solvent. The CMF can be isolated therefrom by e.g. evaporation of such organic extraction solvent to obtain the CMF in pure form to be used in the process of SB Onkarappa, but such evaporation risks causing degradation of the CMF due to thermal stress.

Hence, there was the need for a process that yields 5-(alkoxymethyl)furfural, e.g. 5-(methoxymethyl)furfural and 5-(ethoxymethyl)furfural, from a cellulose or starch source via 5-(chloromethyl)furfural, which process has less risk of causing degradation of the CMF due to thermal stress. It is a desire that the process is such that it is attractive to be carried out on an industrial scale.

It would be preferred that in the process to be designed, there is a convenient possibility to recover part or all of some of the auxiliary materials involved, such as the alcohols and/or the hydrochloric acid.

### Summary of the invention

It has now been found the above objective(s) may be achieved, at least in part, by a process for preparing 5-(alkoxymethyl)furfural from a composition comprising cellulose and/or starch, or a source of cellulose or starch said process comprising the steps of:
a. mixing at a temperature between 10 and 40°C said composition comprising cellulose and/or starch with an aqueous composition comprising hydrochloric acid at a concentration at least 36% to form an aqueous hydrolysate comprising sugars,
b. combining the aqueous hydrolysate with an organic extraction solvent and subject to mixing and heating to a temperature of between 60 and 95°C (preferably between 70 and 85°C) to obtain a mixture comprising organic extraction solvent, 5-(chloromethyl)furfural, and an aqueous liquid,
c. separating the mixture obtained by step b. in a non-aqueous fraction comprising organic extraction solvent and 5-(chloromethyl)furfural and an aqueous fraction comprising water and hydrochloric acid,
d. removing part of the organic extraction solvent from said non-aqueous fraction such that a concentrate is obtained of which the concentration 5-(chloromethyl)furfural is between 35 and 95% by weight,
e. adding an alcohol to the concentrate in a weight ratio alcohol : 5-(chloromethyl)furfural of between 1: 1 and 1 : 10 at a temperature of between 5 and 50°C (preferably between 10 and 40°C) to obtain a mixture comprising 5-(alkoxymethyl)furfural and acetals thereof,
f. removing alcohol and hydrochloric acid from the mixture obtained by step e. to obtain a composition comprising 5-(alkoxymethyl)furfural,
wherein the organic extraction solvent is chosen such that at 20°C less than 2 g of extraction solvent dissolves in 100 ml of the aqueous HCl solution, and wherein the organic extraction solvent is further chosen such that at 20°C at least 5 g of 5-(chloromethyl)furfural can be dissolved in 100 ml of the organic extraction solvent.

### Detailed description of the invention

A key feature of the present invention is that after the phase separation in step c. the organic phase is "concentrated" as to the amount of CMF contained, by removing part of the organic extraction solvent. It was found that a full removal is not needed in order to convert the CMF into the 5-(alkoxymethyl)furfural, which greatly reduces the thermal stress on the CMF, and thus reduces the chance on degradation products form being formed. However, removal of at least part of the organic extraction solvent is preferred so that the total volume that is to handled and subjected to subsequent processing steps is reduced, which brings advantages from a process-economics point of view. Hence, in the present invention, in step d. the amount of organic extraction solvent is removed from said non-aqueous fraction such that a concentrate is obtained of which the concentration 5-(chloromethyl)furfural is between 35 and 95%, preferably between 50 and 95% by weight, more preferably between 65 and 95% by weight.

Although in the process according to the present invention, the conversion of CMF to 5-(alkoxymethyl)furfural can in theory be carried out with various alcohols, both from a process point of view (the conversion), and from the point of view which 5-(alkoxymethyl)furfural components are most desirable for further use, it is preferred that the alcohols in the now claimed process is preferably an aliphatic alcohol, and more preferably such aliphatic alcohol preferably comprises one or more of methanol, ethanol. Most preferably, the alcohol in the presently claimed process is methanol or ethanol. This means that the preferred 5-(alkoxymethyl)furfurals are 5-(methoxymethyl)furfural or 5-(ethoxymethyl)furfural.

Although the process of the present invention focusses on converting CMF, which is still in solution in an organic extraction solvent it was obtained in, to e.g. MMF or EMF, for commercial attractiveness it also matters what happens to side streams and whether auxiliary materials can be recovered. In the process according to the invention, strong hydrochloric acid is used for the hydrolysis / conversion of cellulose or starch or source of either of those, and for economic reasons it is advantageous if at least part of the hydrochloric acid can be recovered. However, the hydrochloric acid is obtained in the process as set out above in admixture with the alcohol used (e.g. methanol or ethanol). From these mixtures it is difficult to recover the HCl in an economically attractive way. It was found, to achieve this, it is beneficial if the alcohol and hydrochloric acid that are removed in step f. of the process of the summary of the invention are combined with water, and the alcohol is separated from aqueous hydrochloric acid.

The stream comprising methanol and HCl, or ethanol and HCl, can be removed from the 5-(alkoxymethyl)furfural product in step f. Preferably, this stream is removed in the form of a gas or vapor from the 5-(alkoxymethyl)furfural product which tends to be liquid. Suitable separation methods are evaporation and distillation. The residual higher boiling fraction, containing 5-(alkoxymethyl)furfural optionally with residual extraction solvent, can be withdrawn from the reactor and processed further. High boiling components are components having boiling points at ambient pressure of higher than 100 °C. Alternatively, the stream of alcohol aqueous hydrochloric acid is separated into alcohol and aqueous hydrochloric acid using a membrane.

To the separated mixture of alcohol and hydrochloric acid obtained in or after step f. water is added. The amount of water tends to be of from 50 to 1 molar equivalent of water relative to the hydrogen chloride present, more specifically of from 20 to 1, more specifically of from 10 to 1, more specifically of from 4 to 1. Next, the aqueous mixture obtained (after adding water to the alcohol / HCl) is separated into aqueous hydrochloric acid and alcohol. Hence, in the present invention, when wishing to recover the hydrochloric acid and/or alcohol, it may be preferred that the alcohol and hydrochloric acid that are removed in step f. are combined with water, and the alcohol is separated from aqueous hydrochloric acid. The separation can be in any way known to the person skilled in the art including but not limited to evaporation and distillation of the alcohol from the aqueous HCl solution. This evaporation preferably is performed such that the methanol and ethanol that is obtained overhead is substantially free from HCl, where substantially free means preferably less than 1 mol% of HCl relative to the molar amount of alcohol. At the same time, the residual aqueous HCl solution is substantially free of methanol and ethanol, substantially free meaning less than 0.1 mol methanol or ethanol per liter aqueous HCl. Following this additional feature, it is possible to recover the methanol or ethanol and the HCl and work such up for recycle back into the CMF conversion step and into the CMF production step, respectively, making such attractive from an economical perspective.

It was found that the process of the present invention can be carried out easily with an aqueous hydrochloric acid solution of about 37 % wt, as such is a commercial strength hydrochloric acid. However, the processes according to the present invention can also be carried out with an aqueous hydrochloric acid composition which is more concentrated. Hence, in the presently claimed process it is preferred that the concentration of the hydrochloric acid in step a. is between 36 and 51 % by weight, preferably between 37 % and 48 %, more preferably between 38 and 46 % by weight.

As to the amount of aqueous hydrochloric acid to be used in the present invention, it is preferred that the composition comprising cellulose and/or starch or source thereof in a. are mixed with the aqueous hydrochloric acid in a weight ratio of cellulose and/or starch : aqueous HCl composition of between 1 : 2 and 1 : 50, preferably between 1 : 5 and 1 : 30.

In the presently claimed process, the reaction of the aqueous hydrochloric acid on the cellulose and/or starch or source thereof can be carried out at a temperature of between 10 and 40°C. Preferably, e.g. for reasons of convenience, this hydrolysis and conversion are carried out at room temperature.

The process according to the invention involves converting sugar molecules to CMF. As to the origin of the sugar molecules various options exist. As mentioned, the presently claimed process utilizes a composition comprising cellulose or source thereof an/or starch or source thereof. As to the cellulose or source thereof, suitable sources may be fibres used e.g. in fabric such as cotton. Hence, in the present invention the cellulose or source thereof is preferably derived from cotton, flax, jute, ramie, hemp, Pina and Abaca). It was found in WO2023/166122 that presence of non-cellulosic material in the form of man-made polymer fibre material, such as polyester and/or polyolefins (polyethylene or polypropylene), polyamide fibre material or elastane fibre material, is not detrimental to the process. This opens up the possibility of using textile waste streams as a source of the cellulose in the presently claimed process. In view of the above, in the present invention it is preferred that the composition comprising cellulose or source thereof comprises cotton fibre. More preferably, the cellulose or source thereof comprising cotton fibre further comprises man-made fibre, preferably polyester and/or polyamide, in an amount of 1 % to 70% by weight, preferably in an amount of 2 to 50% by weight, based on the total of cotton fibre and man-made fibre.

In case in the process according to the present invention the composition comprising cellulose, starch or a source of either of these comprises material that is not hydrolysed by step a. such as non-cellulose/starch material such as lignin or man-made fibre, e.g. polyester, polyamide or other, such non-cellulose is preferably removed at some stage during the process. Hence, in the present invention, in case the composition comprising cellulose or a source thereof comprises lignin or man-made fibre, there is an additional process step between a. and b. of separating such lignin and man-made fibre from the aqueous hydrolysate.

The CMF that is formed by the action of hydrochloric acid on the cellulose and/or starch does dissolve (and or mix, as the melting point of CMF is about 34°C) in the aqueous hydrochloric acid, but is difficult to isolate therefrom and it may also decompose in the strong acid medium. If an organic extraction solvent is present during the CMF production, in which the CMF dissolves easier than in the aqueous hydrochloric acid, and if the organic extraction solvent is selected such that it does not mix well with water or an aqueous liquid, the CMF formed can be extracted from the aqueous hydrochloric acid phase relatively easily during its formation. Hence, in the present invention, both for first and second embodiment, the organic extraction solvent is chosen such that at 20°C less than 2 g (preferably less than 1 g) of extraction solvent dissolves in 100 ml of the aqueous HCl solution, and wherein the organic extraction solvent is further chosen such that at 20°C at least 5 g (preferably at least 10 g) of 5-(chloromethyl)furfural can be dissolved in 100 ml of the organic extraction solvent.

There is a range of potentially suitable organic extraction solvents for this process. A high distribution coefficient (the ratio of the solubility of CMF in the organic phase and the aqueous HCl phase) is desired to maximize CMF extraction and to minimize extraction solvent volume. The solvent should be selected on the basis of price, extraction coefficient, boiling point (preferably above 80°C) stability, etc. Hence, in the process according to the present invention, it is preferred that the organic extraction solvent comprises a halogenated aromatic solvent, diisopropyl ether, ethyl acetate, pentane, hexane, heptane, octane, decane, dodecane, cyclohexane, benzene, toluene, xylene, chloroform, 1,2-dichloroethane, carbon tetrachloride, trichloromethane, and mixtures thereof. Preferably, in case the organic extraction solvent is a halogenated aromatic solvent such is preferably selected from the group consisting of: mono-chlorobenzene, *o-*dichlorobenzene, m-dichlorobenzene, p-dichlorobenzene, 1,2,4-trichlorobenzene, mono-fluorobenzene, *o-*difluorobenzene, m-difluorobenzene, p-diflurorobenzene, 1,2,4-trifluorobenzene, 1,3-dichloro-2-fluorobenzene, fluorotoluene, chlorotoluene, and mixtures thereof, with fluorobenzene being preferred.

As to the amount of organic extraction solvent to be used in step b. of the now claimed process, it is preferred that the aqueous hydrolysate with an organic extraction solvent are combined in a weight ratio aqueous hydrolysate : extraction solvent of between 1 : 0.5 and 1 : 20, preferably between 1:1 and 1 : 10.

As to the temperature at which the interaction between organic extraction solvent and aqueous hydrolysate in b. takes place, it is preferred that in step b. the aqueous hydrolysate is mixed with the organic extraction solvent for at least 30 minutes, preferably at least 50 minutes, and at a the temperature of between 50°C and 110°C, preferably between 50 and 90°C.

In the now claimed process, in step c. the separation in step c. of the mixture obtained by step b. in a non-aqueous fraction comprising organic extraction solvent and 5-(chloromethyl)furfural and an aqueous fraction comprising water and hydrochloric acid can be carried out by any suitable means for separating two immiscible liquid phases, e.g. decanter or centrifuge.

## Claims

1. Process for preparing 5-(alkoxymethyl)furfural from a composition comprising cellulose and/or starch, or a source of cellulose or starch said process comprising the steps of:
a. mixing at a temperature between 10 and 40°C said composition comprising cellulose and/or starch with an aqueous composition comprising hydrochloric acid at a concentration at least 36% to form an aqueous hydrolysate comprising sugars,
b. combining the aqueous hydrolysate with an organic extraction solvent and subject to mixing and heating to a temperature of between 60 and 95°C to obtain a mixture comprising organic extraction solvent, 5-(chloromethyl)furfural, and an aqueous liquid,
c. separating the mixture obtained by step b. in a non-aqueous fraction comprising organic extraction solvent and 5-(chloromethyl)furfural and an aqueous fraction comprising water and hydrochloric acid,
d. removing part of the organic extraction solvent from said non-aqueous fraction such that a concentrate is obtained of which the concentration 5-(chloromethyl)furfural is between 35 and 95% by weight,
e. adding an alcohol to the concentrate in a weight ratio alcohol : 5-(chloromethyl)furfural of between 1: 1 and 1 : 10 at a temperature of between 5 and 50°C to obtain a mixture comprising 5-(alkoxymethyl)furfural and acetals thereof,
f. removing alcohol and hydrochloric acid from the mixture obtained by step e. to obtain a composition comprising 5-(alkoxymethyl)furfural,
wherein the organic extraction solvent is chosen such that at 20°C less than 2 g of extraction solvent dissolves in 100 ml of the aqueous HCl solution, and wherein the organic extraction solvent is further chosen such that at 20°C at least 5 g of 5-(chloromethyl)furfural can be dissolved in 100 ml of the organic extraction solvent.

2. Process according to claim 1, wherein the alcohol is an aliphatic alcohol, preferably the alcohol comprises one or more of methanol, ethanol, more preferably the alcohol is methanol or ethanol.

3. Process according to claim 1 or 2, wherein, in case the composition comprising cellulose or a source thereof comprises lignin or man-made fibre, there is an additional process step between a. and b. of separating such lignin and man-made fibre from the aqueous hydrolysate.

4. Process according to any of the preceding claims, wherein the alcohol and hydrochloric acid that are removed in step f. are combined with water, and the alcohol is separated from aqueous hydrochloric acid.

5. Process according to any of the preceding claims, wherein composition comprising cellulose and/or starch in a. are mixed with the aqueous hydrochloric acid in a weight ratio of cellulose and/or starch : aqueous HCl composition of between 1 : 2 and 1 : 50, preferably between 1 : 5 and 1 : 30.

6. Process according to any of the preceding claims, wherein the composition comprising cellulose or source thereof comprises cotton fibre.

7. Process according to claim 6, wherein the composition comprising cotton fibre further comprises man-made fibre, preferably polyester and/or polyamide, in an amount of 1 % to 70% by weight, preferably in an amount of 2 to 50% by weight, based on the total of cotton fibre and man-made fibre.

8. Process according to any of the preceding claims, wherein the concentration of the hydrochloric acid in step a. is between 36 and 51% by weight, preferably between 37% and 48%, more preferably between 38 and 46% by weight.

9. Process according to any of the preceding claims, wherein in step b. the aqueous hydrolysate with an organic extraction solvent are combined in a weight ratio aqueous hydrolysate : extraction solvent of between 1 : 0.5 and 1 : 20, preferably between 1:1 and 1:10.

10. Process according to any of the preceding claims, wherein in step b. the aqueous hydrolysate is mixed with the organic extraction solvent for at least 30 minutes and at a the temperature of between 50°C and 110°C.

11. Process according to any of the preceding claims, wherein in step d. such an amount of organic extraction solvent is removed from said non-aqueous fraction, that a concentrate is obtained of which the concentration 5-(chloromethyl)furfural is between 50 and 95% by weight, preferably between 65 and 95% by weight.

12. Process according to any of the preceding claims, wherein the organic extraction solvent comprises a halogenated aromatic solvent, diisopropyl ether, ethyl acetate, pentane, hexane, heptane, octane, decane, dodecane, cyclohexane, benzene, toluene, xylene, chloroform, 1,2-dichloroethane, carbon tetrachloride, trichloromethane, and mixtures thereof.

13. Process according to claim 12, wherein the wherein the halogenated aromatic extraction solvent is selected from the group consisting of: mono-chlorobenzene, o-dichlorobenzene, m-dichlorobenzene, p-dichlorobenzene, 1,2,4-trichlorobenzene, mono-fluorobenzene, o-difluorobenzene, m-difluorobenzene, p-diflurorobenzene, 1,2,4-trifluorobenzene, 1,3-dichloro-2-fluorobenzene, fluorotoluene, chlorotoluene, and mixtures thereof.
